# EUROPÄISCHE PATENTANMELDUNG

(11) **EP 2 143 575 A1**
(43) Veröffentlichungstag der Anmeldung: **13.01.2010**
(21) Anmeldenummer: 09164776.8
(22) Anmeldetag: 07.07.2009
(51) Int. Cl.: B60H 3/00, A61L 9/12

(54) **Duftmittelspender**

(30) Priorität: 11.07.2008 DE 102008032613
(71) Anmelder: Behr GmbH & Co. KG, 70469 Stuttgart (DE)
(72) Erfinder: Kroner, Peter, 66482 Zweibrücken (DE); Fritsche, Uwe, 71686 Remseck (DE); Zeller, Kuno, 70435 Stuttgart (DE); Stiehler, Daniela, 70597 Stuttgart (DE); Fieger, Martin, 71636 Ludwigsburg (DE); Fischle, Klaus, 71732 Tamm (DE); Lochmahr, Karl, 71665 Vaihingen/Enz (DE); Rais, Thomas, 71672 Marbach/Neckar (DE); Treier, Joachim, 77728 Oppenau (DE)

(57) **Zusammenfassung**

Bei einer Beduftungsvorrichtung (1, 21, 37, 68) wird eine austauschbare Duftmittelvorratsvorrichtung (2, 22, 37, 69) vorgesehen. Die Duftmittelvorratsvorrrichtung (2, 22, 37, 69) weist eine Duftmittelbehältereinrichtung (5, 28, 39, 41, 57, 69), eine Duftmittelaustragseinrichtung (6, 25, 46, 64, 72) und eine Koppelungseinrichtung (15, 49, 73) zur Ankoppelung an wenigsten eine Akfuatoreinrichtung (17, 35, 56, 74) auf.

## Beschreibung

Die Erfindung betrifft eine Duftmittelvorratsvorrichtung, welche wenigstens eine Duftmitteibehältereinrichtung und wenigstens eine Duftmittelaustrageeinrichtung aufweist. Weiterhin betrifft die Erfindung eine Beduftungsvornchtung, insbesondere eine Beduftungsvorrichtung für ein Fahrzeug, vorzugsweise eine Beduftungsvorrichtung für ein Kraftfahrzeug.

Bei der Aufarbeitung von Luft erfolgt in zunehmendem Maße auch ein Einbringen von Duftstoffen in die aufzuarbeitende bzw. in die aufgearbeitete Luft. Duftstoffe werden nämlich über das Limbische System unter Umgehung des Bewusstseins wahrgenommen. Aufgrund dieser Umgehung von Denkprozessen kann mittels Duftstoffen besonders effektiv auf den Gemütszustand einer Person eingewirkt werden. Dies wird beispielsweise bei der Klimatisierung von Büros, Verkaufsstätten und Fahrzeugen, insbesondere von Luftfahrzeugen, ausgenutzt, um beispielsweise die Kauflust zu fördern, oder nervöse Passagiere zu beruhigen, Insbesondere werden die Duftstoffe zur Schaffung einer Atmosphäre und zur Steuerung des Wohlbefindens eingesetzt. Auch im Kraftfahrzeugbereich wird eine Beduftung in zunehmendem Maße eingesetzt.

Bislang werden im Kraftfahrzeugbereich zur Beduftung des Fahrzeuginnenraums oftmals Duftbäume aus Karton oder einem entsprechenden Material verwendet. Diese Duftbäume sind mit einem Duftstoff getränkt, der sukzessive an die Außenluft abgegeben wird. Die Duftbäume werden in der Regel am Fahrzeuginnenspiegel befestigt, wo sie gut erkennbar sind, Die Duftbäume werden Jedoch oftmals als kitschig und störend empfunden. Darüber hinaus geben die Duftbäume den Duftstoff kontinuierlich an die Außenluft ab, so dass sich die Fahrzeuginsassen nach einiger Zeit an den Geruch gewöhnen und ihn schlussendlich nicht mehr wahrnehmen. Manche Personen bevorzugen jedoch eine dauerhaft wahrnehmbare Beduftung des Fahrzeuginnenraums.

Um eine Gewöhnung an den Duftstoff zu verhindern, wurden bereits Beduftungsvorrichtungen vorgeschlagen, bei denen der Duftstoff stoßweise in Intervallen freigesetzt wird. Diese Duftstöße können grundsätzlich über einen beliebig langen Zeitraum bewusst wahrgenommen werden.

Komplexe Beduftungsvorrichtungen werden aus Kostengründen in der Regel nachfüllbar ausgeführt. Das heißt, dass ein großer Anteil der Baugruppen der Beduftungsvorrichtung dauerhaft verwendet wird. Die sich verbrauchenden Duftstoffe werden entweder mit einer Nachfülleinheit (meist eine Nachfüllspritze) nachgefüllt, oder der leere Duftmittelbehälter wird durch einen neuen Duftmittelbehälter ersetzt. Es hat sich jedoch herausgestellt, dass ein derartiges Nachfüllen einer Beduftungsvorrichtung aus hygienischen Gründen problematisch ist. Denn bei einem Nachfüllen des Duftmittels bzw. bei einem Austausch des Duftmittelbehälters können Verunreinigungen, Sporen oder Keime in das Duftmittel gelangen und sich dort ggfs, vermehren. Diese verteilen sich anschließend mit dem Duftmittel im Kraftfahrzeuginnenraum, wo sie in die Atemwege der Fahrzeuginsassen gelangen. Da die Fahrzeugpassagiere den Duftstoffen funktionsbedingt über einen langen Zeitraum ausgesetzt sind, ist dies aus medizinischer Sicht nicht unbedenklich.

Möglich ist es, den Duftstoffen eine entsprechende Menge an Konservierungsstoffen beizumischen. Jedoch können sich auch derartige Konservierungsstoffe als gesundheitlich bedenklich erweisen. Darüber hinaus lehnen viele Verbraucher Konservierungsstoffe aus grundsätzlichen Erwägungen ab. Möglich ist es auch, die Duftstoffe vor dem Verteilen (bzw. beim Verteilen) durch Erhitzen zu sterilisieren. Hier erweist sich jedoch als nachteilig, dass die Duftsuthentizstät der Duftstoffe beim Erhitzen oftmals leidet.

Die Aufgabe der Erfindung liegt somit darin, eine verbesserte Duftmittelvorratsvorrichtung, bzw. eine verbesserte Beduftungsvorrichtung vorzuschlagen.

Dazu wird vorgeschlagen, eine Duftmittelvorratsvorrichtung, welche wenigstens eine Duftmittelbehältereinrichtung und wenigstens eine Duftmittelaustragseinrichtung aufweist, mit wenigstens einer Koppelungseinrichtung zur Ankopplung an wenigstens eine Akutatoreinrichtung zu versehen. Die Duftmittelvorratsvorrichtung kann als austauschbare Einheit aus Duftmittelbehältereinrichtung, Duftmittelaustragselnrichtung und Koppelungseinrichtung ausgebildet werden, Wenn also beispielsweise das Duftmittel einer Beduftungsvorrichtung erschöpft ist, so kann die gesamte Dufmittelvorratsvorrichtung ausgetauscht werden. Dadurch kann insbesondere eine Kontamination des Duftmittels beim Nachfüllen von Duftmittel verhindert werden, oder zumindest stark verringert werden. Dadurch, dass lediglich eine Koppelungseinrichtung zur Ankopplung der Duftmittelvorratsvorrichtung an wenigstens eine Aktuatorvorrichtung vorgesehen werden kann, steigt der bauliche Aufwand der Duftmittelvorratsvorrichtung üblicherweise nicht übermäßig an. Üblicherweise ist nämlich gerade die Akutatoreinrichtung relativ komplex und aufwändig. Diese kann jedoch unabhängig von der Duftmittelvorratsvorrichtung vorgesehen werden, so dass diese nicht mitsamt der Duftmittelvorratsvorrichtung ausgetauscht werden muss und daher dauerhaft verwendet werden kann. Auch die Duftmittelaustragseinrichtung kann üblicherweise einfach ausgebildet werden, so dass auch hier die Kosten bei einem Austausch der gesamten Duftmitteivorratsvorrichtung nicht übermäßig ansteigen müssen.

Alternativ oder zusätzlich wird vorgeschlagen, eine Duftmitteivorratsvorrichtung mit zumindest einem Informationsspeichermittel zu versehen, welches insbesondere als elektronisches Informationsspeichermittel ausgebildet ist. Bei den gespeicherten Informationen kann es sich beispielsweise um die Art des Duftstoffs, um die Füllmenge, um das Herstellungsdatum, um das Verfallsdatum, um eine Chargennummer, um die Duftintensität des Duftmittels, um eine sonstige Stoffcharakteristik des Duftmittels, um sonstige Produktinformationen, um den Hersteller und/oder um Dosierungshinweise handeln. Als Informationsspeichermittel können zum Beispiel einfache Papieretiketten, aber auch beispielsweise elektronische Chips oder sonstige elektronische, magnetische und/oder optische Speichermedien eingesetzt werden. Insbesondere ist es auch möglich, dass die im Informationsspeichermittel gespeicherten Informationen während des Betriebs der Duftmittelvorratsvorrichtung bzw. während des Betriebs der Beduftungsvorrichtung, in die die Duftmittelvorratsvorrichtung eingesetzt ist, geändert werden. Hierbei kann es sich insbesondere um einen Befüllungszustand der Duftmittelbehältereinrichtung, um die Anzahl der Betriebsstunden, um ein Einsatzprofil, um die Einsatzhistorie und/oder um einen Einsatzort (z.B. basierend auf Daten, die von einem Navigationssystem zur Verfügung gestellt werden) handeln. Die Informationen können derart angezeigt werden, dass sie von einem Menschen direkt gelesen werden können. Dies ist beispielsweise der Fall, wenn es sich bei dem Informationsspeichermittel um ein einfaches Papieretikett handelt. Zur Erkennung des Füllstands ist es auch möglich, die Duftmittelbehältereinrichtung mit einem Fenster zu versehen und/oder zumindest teilweise aus einem transparenten bzw. durchsichtigen Material zu feigen. Es kann sich jedoch auch als sinnvoll erweisen, wenn die Informationen von einem entsprechenden Informationsübertragungsmittel ausgelesen werden, und die von dem Informationsübertragungsmittel ausgelesenen Informationen für eine automatische Steuerung genutzt werden bzw. von dem Informationsübertragungsmittel mittelbar an eine Person weitergeleitet werden. Bevorzugt handelt es sich bei dem Informationsübertragungsmittel um ein elektronisches Informationsübertragungsmittel. Ein derartiges mechanisiertes Auslesen bzw. Übertragen von Informationen kann beispielsweise durch eine optische Kamera, einen Barcode, einen 2-D Code, einen RFID-Chip (RFID für Radio Frequency IDentification), durch eine Bluetooth-Übertragung oder durch kontaktbehaftete elektrische Signale erfolgen. Insbesondere ist es auch denkbar, dass eine Informationsübertragung nicht nur unidirektional, sondern insbesondere auch bidirektional erfolgt. Z.B. können auf diese Weise Ortskoordinaten eines Navigationssystems an die Duftmittelvorratsvorrichtung Übertragen werden. Die auf dem Informationsspeichermittel gespeicherten Informationen können auch - zumindest teilweise - in verschlüsselter Form gespeichert sein. Dadurch ist es besonders gut möglich, die Produktauthentizität sicherzustellen und Fälschungen zu erschweren. Darüber hinaus ist es auch möglich, gewisse Informationen zu speichern, die vom Benutzer nicht aktiv gelesen und/oder verändert werden können sollen. Beispielsweise kann das Benutzerverhalten (Anzahl der Eingriffe, Öffnen der Beduftungsvorhchtung, Zahl der Einschaltvorgänge, Betriebsstunden usw.) gespeichert werden, um nötigenfalls bei einem Garantiefall ausgelesen und verwendet werden zu können.

Es kann sich als vorteilhaft erweisen, wenn wenigstens eine Koppelungseinrichtung zumindest teilweise als mechanische Koppelungseinrichtung, zumindest teilweise als magnetische Koppelungseinrichtung, zumindest teilweise als elektrostatische Koppelungseinnchtung, zumindest teilweise als Fluiddruckeinrichtung und/oder zumindest teilweise als akustische Koppelungseinrichtung ausgeführt ist. Mit Hilfe derartiger Koppelungseinrichtungen kann die Betätigung der Duftmittelvorratsvorrichtung (insbesondere der Duftmittelaustragseinrichtung) auf besonders einfache, verschieißarme, kostengünstige und/oder beschädigungsunempfindliche (insbesondere beim Austausch der Duftmittelvorratsvorrichtung) Weise ausgeführt weiden. Eine mechanische Koppelung kann beispielsweise durch einen Stößel erfolgen. Eine magnetische bzw. elektrostatische Koppelungseinnchtung kann im einfachsten Fall durch eine Nebeneinanderanordnung von Akutatoreinrichtung und Duftmitteiaustragseinnchtung realisiert werden. Die magnetischen bzw. elektrostatischen Kräfte können dann einfach über einen Luftspalt übertragen werden. Möglich ist es jedoch auch, ein die Koppelung verstärkendes Bauteil zu verwenden. Im Falle einer magnetischen Koppelungseinrichtung könnte beispielweise ein die Magnetfeldlinien leitender Kern aus einem weichmagnetischen Material vorgesehen werden. Eine akustische Koppelungseinrichtung kann insbesondere in Form eines mechanischen Kontakts entsprechender Bauteile realisiert werden. Bei der akustischen Koppelung kann es sich nicht nur um Frequenzen handeln, welche in hörbaren Bereich liegen. Vielmehr kann beispielsweise auch Ultraschall oder Infraschall genutzt werden. Bei der Fluiddruckeinrichtung kann es sich um eine Druckluftpumpe handeln, die die Duftmittelbehältereinrichtung beispielsweise mit Druckluft beaufschlägt. Die Beaufschlagung mit Druckluft kann dabei durch eine Zufuhr von Druckluft in den eigentlichen Behälter, der das Duftmittel aufweist, hinein erfolgen. Möglich ist es aber auch, dass der eigentliche Behälter, der das Duftmittel aufweist, beispielsweise verformbar oder elastisch ausgebildet ist und von außen mittels der Druckluft unter Druck gesetzt wird. Auch ist es möglich, dass das innere der Duftmittelbehältereinrichtung bereits ab Werk unter Druck gesetzt wurde, beispielsweise mit Hilfe von Druckluft oder mit Hilfe von Treibgas.

Vorteilhaft ist es, wenn die Duftmittelvorratsvorrichtung, insbesondere zurmindest eine Duftmittelbehältereinrichtung, luftdicht oder keimdicht ausgeführt ist. Auf diese Weise kann ein problematisches Verunreinigen und ggfs. Verkeimen des Duftmittels vermieden werden. Dies ist aus gesundheitlichen Erwägungen im Verhältnis zu Nachfüllsystemen, bei dem es zu einer Verunreinigung oder einem Verkeimen des Duftmittels kommen kann, vorzuziehen.

Es ist möglich, die Duftmittelvorratsvorrichtung, insbesondere zumindest eine Duftmittelbehältereinrichtung und/oder zumindest eine Duftmittelaustragseinrichtung zumindest teilweise wieder befüllbar und/oder zumindest teilweise rezyklierbar auszuführen. In diesem Zusammenhang ist insbesondere an eine Wiederbefüllung bzw. eine Rezyklierung beim Hersteller der Duftmittelvorratsvorrichtung, bzw. bei einem Service partner des Herstellers der Duftmittelvorratsvorrichtung zu denken. Dort ist es nämlich möglich, die Wiederbefüllung der Duftmittelvorratsvorrichtung unter sterilen Bedingungen durchzuführen. Dadurch können einerseits Kosten gespart werden, andererseits kann eine aus hygienischer Sicht unbedenkliche Beduftungsvornchtung ermöglicht werden. Die Duftmittelvorratsvorrichtung kann dann beispielsweise als bepfandetes Bauteil in Umlauf gebracht werden.

Möglich ist es, dass zumindest eine Duftmittelaustragseinrichtung zumindest eine Zerstäubereinrichtung und/oder eine Pumpenzerstäubereinrichtung aufweist. Derartige Baugruppen haben sich für die Zerstäubung von Flüssigkeiten bewährt und sind darüber hinaus in der Regel kostengünstig verfügbar. Bei einer Zerstäubereinrichtung kann es sich im Übrigen auch um eine Art Zerstäubungsdüse handeln. Um eine Flüssigkeit mit einer derartigen Zerstäuberdüse zerstäuben zu können, muss die Flüssigkeit üblicherweise mit einem erhöhten Druck zugeführt werden. Ein derartiger erhöhter Druck kann beispielsweise durch eine Druckbeaufschlagung der Duftmittelbehältereinrichtung ab Werk oder durch Vorsehen einer kleinen Pumpe (z.B. einem Pumpzerstäuberkopf) realisiert werden. Auch eine externe Fluiddruckbeaufschlagung der Duftmittelbehältereinrichtung ist selbstverständlich möglich. Besonders vorteilhaft ist es wenn, die Duftmittelaustragseinrichtung Tröpfchen mit einer Größe von 3 bis 12 Mikrometer, insbesondere im Bereich von 5 Mikrometer austrägt. Im Zusammenhang mit einer Duftmittelaustragvorrichtung kann gegebenenfalls auch eine Prallplatte für die ausgetragene Flüssigkeit bzw, den ausgetragenen Flüssigkeitsnebel vorgesehen werden, um eine nochmals gleichmäßigere Vermischung von Luft und Duftmittel zu realisieren. Da die Prallplatte in der Regel mit ausgetragenen Flüssigkeitströpfchen in Kontakt kommt, ist es aus hygienischen Gründen sinnvoll, wenn die Prallplatte ebenfalls mitsamt der Duftmittelbebältervorrichtung ausgetauscht wird. In einer weiteren Variante kann die Prallplatte auch mit einer Heizeinrichtung, insbesondere einer elektrischen Heizeinrichtung versehen oder als solche ausgebildet sein.

Ebenfalls kann es sich als sinnvoll erweisen, wenn die Duftmittelvorratsvorrichtung wenigstens ein Verrastmlitel zur Verrastung in einem Aufnahmebereich für die Duftmittelvorratsvorrichtung aufweist. Dabei kann es sich beispielsweise um Nuten, hinterschnittene Vorsprünge oder dergleichen handeln. Als besonders sinnvoll kann es sich auch erweisen, wenn die Duftmittelvorratsvorrichtung eine Führungsnut, einen vorspringenden Stift und/oder ein Federmittel für ein sogenanntes Push-Push-System aufweist. De Duftmittelvorratsvorrichtung kann dann durch einfaches Eindrücken in ihrem dafür vorgesehenen Aufnahmebereich verrastet werden, und durch nochmaliges Drücken wieder entriegelt werden, um anschließend aus dem Aufnahmebereich wieder entnommen werden zu können. Derartige Push-Push-Systeme sind insbesondere von Kugelschreibern her bekannt.

Möglich ist es auch, die Duftmittelvorratsvorrichtung mit wenigstens einem integral ausgebildeten Luftkanalbereich auszuführen. Der Luftkanalbereich ist dabei bevorzugt in einem Bereich angeordnet, in dem Duftmitteltröpfchen, die von der Duftmittelaustragseinrichtung freigegeben wurden, an eine Luftkanalwand prallen können. Auch hier kann eine Kontaminierung des Luftstroms verringert werden, da Bauteile, die mit flüssigem Duftmittel in Kontakt treten können, durch einen Austausch einer Duftmittelvorratsvorrichtung ebenfalls mit ausgetauscht werden. Auch kann die Gefahr einer Duftverschleppung bei einem Duftwechsel verringert werden, da gegebenenfalls noch an der Luftkanalwand anhaftende Duftmittelstoffe durch den Wechsel der Duftmittelvorratsvorrichtung entfernt werden. Üblicherweise handelt es sich bei dem Luftkanalbereich um geringwertige Bauteile, so dass der Materialaufwand mit der vorgeschlagenen Bauausführung nicht übermäßig anwächst.

Weiterhin wird vorgeschlagen, die Duftmittelvorratsvorrichtung mit wenigstens einer Duftmittelbehandlungseinrichtung für ausgetragenes Duftmittel zu versehen. Die Duftmittelbehandlungseinrichtung kann dabei insbesondere als Heizeinrichtung und/oder als Luftverwirbelungseinrichtung ausgebildet sein. Durch eine solche Duftmittelbehandlungseinrichtung kann gegebenenfalls auf einen Wandbereich auftreffende Flüssigkeit vorteilhaft in den gasförmigen Zustand überführt werden und/oder es kann von vornherein verhindert werden, dass es überhaupt zu einem (größeren) Niederschlag von Flüssigkeit an einer Begrenzungswand eines luftführenden Kanals kommt. Auch hierdurch können Verkeimungen und Duftverschleppungen verhindert oder zumindest verringert werden. Auch ist es möglich, die Effektivität der Beduftungsvorrichtung zu steigern.

Weiterhin wird eine Beduftungsvorrichtung vorgeschlagen, die wenigstens eine Duftmittelvorratsvorrichtung mit dem oben beschriebenen Aufbau aufweist. Die Beduftungsvorrichtung ist dabei insbesondere als Beduftungsvorrichtung für ein Fahrzeug, vorzugsweise als Beduftungsvorrichtung für ein Kraftfahrzeug ausgebildet. Vorzugsweise ist zumindest eine Duftmittelvorratsvorrichtung der Beduftungsvornchtung austauschbar ausgeführt. Bei dem Fahrzeug (Kraftfahrzeug) kann es sich sowohl um ein Wasserfahrzeug, ein Luftfahrzeug, als auch um ein Landfahrzeug (schienengebunden/nichtschienengebunden) handeln. Eine derartige Beduftungsvorrichtung kann entsprechend der obigen Ausführungen weitergebildet werden. Die Beduftungsvoriichtung weist dann die bereits beschriebenen Vorteile und Eigenschaften in analoger Weise auf.

Alternativ oder zusätzlich wird vorgeschlagen, die Beduftungsvorrichtung mit wenigstens einem Informationsübertragungsmittel zu versehen. Mit Hilfe eines derartigen Informationsübertragungsmittels kann beispielsweise die in einem Informationsspeichermittel gespeicherte Information automatisiert ausgelesen werden. Die derart gewonnene Information kann anschließend von der Beduftungsvornchtung für die Beduftung des Fahrzeuginnenraums benutzt werden, bzw. an einen Fahrzeuginsassen weitergegeben werden. Das Informationsübertragungsmittel ist dabei bevorzugt als elektronisches Informationsübertragungsmittel ausgebildet. Das Infolrmationsübertragungsmittel kann beispielsweise eine optische Kamera, einen Barcodescanner, einen 2-D Code-Scanner, eine RFID-Übertragungsstrecke, eine Bluetooth-Übertrageungsvorrichtung und/oder eine kontaktbehaftete elektrische Signal-übertragungseinrichtung aufweisen. Insbesondere ist es auch denkbar, dass eine Informationsübertragung nicht nur unidirektional, sondern insbesondere auch bidirektional erfolgt. Die übertragenen Informationen, bzw. Informationen, welche auf den übertragenen Informationen basieren, können dem Benutzer der Vorrichtung über ein beliebiges Informations darstellungsmittel, wie beispielsweise über ein Display, über Anzeigeleuchtumittel, über eine akustische Rückmeldung usw. dargestellt werden. Möglich ist es im Übrigen auch, dass die Datenübertragung des Informationsübertragungsmittels zumindest teilweise und/oder zumindest zeitweise verschlüsselt erfolgt. Auch dadurch kann die Produktsicherheit bzw. die Sicherheit gegenüber unzulässigen Manipulationen nochmals gesteigert werden.

Es kann sich als sinnvoll erweisen, die Beduftungsvorrichtung mit wenigstens einem Aufnahmeraum und/oder wenigstens einem Verrastmittel zur Aufnahme wenigstens einer Duftmittelvorratsvorrichtung zu versehen. Dies kann auf unterschiedliche Weise geschehen Insbesondere können die Aufnahmeräume als eine Art Schublade ausgeführt werden, in die die Duftmittelvorratsvorrichtung(en) eingelgt werden kann (können). Auch können die Duftmittelvorratsvorrichtungen als Ganzes in den Aufnahmeraum der Beduftungsvorrichtung eingeschraubt werden, über ein zusätzliches Bauteil verclipst werden, oder über eine Kippbewegung im Aufnahmeraum der Beduftungsvorrichtung verankert werden. Besonders vorteilhaft kann es jedoch sein, den Aufnahmeraums sowie das Verrastmittel der Beduftungsvorrichtung derart auszubilden, dass es mit einem Verrastmittel einer Duftmittelvorratsvorrichtung derart zusammenwirkt, dass sich eine Push-Push-Lösung zum Arretieren und anschließenden Lösen der Duftmittelvorratsvorrichtung im dazu korrespondierenden Aufnahmeraum der Beduftungsvornchtung ergibt. Zusätzlich kann ein weiterer Sicherheitsmechamsmus zum Schutz vor einer unbeabsichtigten Entnahme, insbesondere eine Kindersicherung vorgesehen sein.

Im Weiteren ist es möglich, die Beduftungsvorrichtung mit wenigstens einer Luftbeeinflussungsvorrichtung zu versehen, insbesondere mit wenigstens einer Luftfördereinrichtung, wenigstens einer Lufttemperaturänderungseinrichtung und/oder wenigstens einer Luftverwirbelungseinrichtung. Bei der Luftfördereinrichtung kann es sich beispielsweise um einen Vehtilator bzw. um ein Gebläse handeln. Dadurch können ein variabler Luftdurchsatz, und damit eine speziellen Senutzervorgaben genügende Seduftung des Fahrzeuginnenraums erfolgen, Möglich ist es jedoch auch, die Luftbeeinflussungsvorrichtung beispielsweise als Luftleitelement, wie insbesondere als bewegliche Luftleitklappe auzubilden. Mit einer derartigen Luftleitklappe ist es beispielsweise beim Vorhandensein mehrerer Duftmittekorratsvorrichtungen möglich, den die Beduftungsvorrichtung durchströmenden Luftstrom selektiv zur aktiven Duftmittelvorratsvorrichtung zu lenken. Dadurch kann die Beduftungsleistung erhöht werden, der erforderliche Luftstrom verringert werden und insbesondere die Duftauthentizität erhöht werden, da die Gefahr einer Duftverschleppung verringert werden kann. Die Stellung der Luftleitklappe kann insbesondere in Abhängigkeit der Duftauswahl gewählt werden. Weiterhin kann eine Lufttemperaturänderungseinrichtung, wie beispielsweise ein Heizelement oder ein Kühlelement (beispielsweise ein Wärmetauscher) vorgesehen werden. Durch derartige Vorrichtungen kann die aus der Beduftungsvorrichtung austretende Luft auf das gleiche Temperaturniveau gebracht werden, wie es im Fahrzeuginneren vorherrscht. Darüber hinaus ist es möglich, dass beispielsweise durch ein Erwärmen der Luft die Intensität des Dufts gesteigert bzw. vergleichmäßigt (Duftauthentizität) wird. Bei einer Luftverwirbelungseinrichtung kann es sich um Luftleitelemente, Prallplatten oder ähnliche Vorrichtungen handeln. Mit Hilfe einer derartigen Luftverwirbelung kann eine besonders gleichmäßige Verteilung des Duftstoffs im Luftstrom nochmals gefördert werden.

Es kann sich als vorteilhaft erweisen, wenn zumindest eine Aktuatorvorrichtung zumindest teilweise als Motoreinrichtung, zumindest teilweise als Magneteinrichtung, zumindest teilweise als elektrostatische Einrichtung, zumindest teilweise als fluiddruckerhöhende Einrichtung und/oder zumindest teilweise als Bimetalleinrichtung ausgebildet ist. Mit Hilfe einer geeigneten, zu einer Duftmittelaustragseinrichtung der Duftmittelvorratsvomchtung korrespondierend ausgebildeten Akutatoreinrichtung, kann die Beduftungsvorrichtung beispielsweise einen gesteuerten, variablen, stoßweisen und/oder intervallartigen Duftmittelaustrag erzeugen. Die Zeiträume, die zwischen zwei Duftstößen liegen, können beispielsweise zwischen 1 und 2 Minuten liegen. Die Länge der einzelnen Duftstöße beträgt vorzugsweise zwischen 0,1 und 10 Sekunden. Es kann vorgesehen werden, dass die Länge der Zeitdauern zwischen einzelnen Duftstößen und/oder die Länge der einzelnen Duftstöße vom Benutzer eingestellt werden kann. Dies kann einerseits völlig frei, aber auch mittels fest vorgegebener Stufungen realisiert werden, die vom Benutzer ausgewählt werden können. Dafür können geeignete Bedienelemente vorgesehen werden.

Möglich ist es auch, dass die Beduftungsvorrichtung mit einer Mehrzahl von Aufnahmeräumen zur Aufnahme von Duftmittelvorratsvorrichtungen ausgebildet wird. Vorzugsweise sind aus Platzgründen nicht mehr als fünf Aufnahmeräume zur Aufnahme von Duftmittelvorratsvorrichtungen vorgesehen. Eine sinnvolle Anzahl kann sich aber auch mit zwei, drei und/oder vier verschiedenen Aufnahmeräumen ergeben. Vorzugsweise ist das System so aufgebaut, dass vom Benutzer immer nur ein einzelner Duft angewählt werden kann. Dazu können geeignete Bedienelemente vorgesehen werden. Sofern die in die Aufnahmeräumen eingelegten Duftmittelvorratsvorrichtungen Grundduftrichtungen enthalten, kann es aber auch ermöglicht werden, zwei oder mehrere Düfte gleichzeitig auszuwählen, um so eine Vielzahl von Duftkompositionen zu ermöglichen. Eine derartige Mischstellung kann beispielsweise dann freigegeben werden, wenn das Informationsübertragungsmittel festgestellt hat, dass sich in den Duftmittelvorratsvorrichtungen Duftmittel mit Grundduftrfchtungen befinden, die sinnvoll miteinander vermischt werden können.

Im Folgenden wird die Erfindung unter Verwendung einzelner ausgewählter Ausführungsbeispiele und unter Verweis auf die beigefügten Figuren näher beschrieben Es zeigen:
- Fig. 1:: Ein erstes Ausführungsbeispiel einer Beduftungsvorrichtung mit einer Duftmittelkartusche in schematischer, perspektivischer Ansicht;
- Fig. 2:: ein zweites Ausführungsbeispiel einer Beduftungsvorrichtung mit einer Duftmittelkartusche in schematischer Querschnittsansicht;
- Fig. 3:: ein drittes Ausführungsbeispiel einer Beduftungsvorrichtung mit zwei Duftmittelkartuschen in schematischer Draufsicht von oben;
- Fig. 4:: eine Teilansicht des in Fig. 3 gezeigten dritten Ausführungsbeispiels in schematischer, perspektivischer Ansicht;
- Fig. 5:: die im dritten Ausführungsbeispiel verwendete Duftmittelkartusche in schematischer, perspektivischer Ansicht;
- Fig.6:: ein zweites Ausführungsbeispiel für eine Duftmittelkartusche in schematischer Querschnittansicht;
- Fig. 7:: ein viertes Ausführungsbeispiel für eine Beduftungsvorrichtung mit einem dritten Ausführungsbeispiel einer Duftmittelkartusche im schematischen Querschnitt.

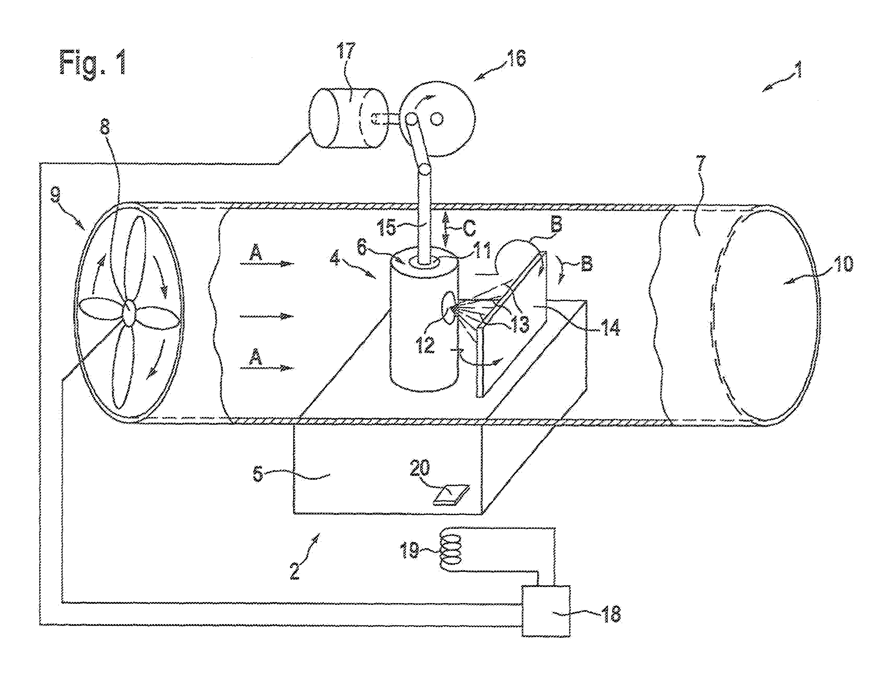

Fig. 1 zeigt in perspektivischer Sichtweise eine schematische Ansicht einer Beduftungsvorrichtung 1, welche eine Duftmittelkartusche 2 aufweist. Die Duftmittelkartusche 2 weist in ihrem unteren Bereich 3 einen Duftmittelbehälter 5 auf, in dem ein Duftmittel enthalten ist, dass zur Beduftung eines Kraftfahrzeuginnenraums verwendet werden soll. In einem oberen Bereich 4 weist die Duftmittelkartusche 2 einen Pumpzerstäuberkopf 6 auf. Pumpzerstäuberkopf 6 und Duftmittelbehälter 5 sind als integrale Einheit ausgeführt.

Der obere Bereich 4 der Duftmittelkartusche 2 befindet sich in einem Luftkanal 7 der Beduftungsvorrichtung 1. Im Luftkanal 7 ist an einer Ansaugseite 9 ein Gebläse 8 vorgesehen. Das Gebläse 8 fördert die zu bedüftende Luft in Richtung der Pfeile A am Pumpzerstäuberkopf 6 vorbei zur Ausblasöffnung 10 des Luftkanals 7. Die Ansaugöffnung 9 sowie die Ausblasöffnung 10 können dabei mit weiteren luftführenden Kanälen verbunden sein. Beispielsweise kann die Beduftungsvorrichtung 1 als integraler Teil einer Kraftfahrzeugklimaanlage verwendet werden, oder in einen Luftführungskanal des Belüftungssystems eines Kraftfahrzeugs eingesetzt werden.

Die Beduftungsvorrichtung 1 ist derart ausgebildet, dass es möglich ist, die Duftmittelkartusche 2 als Einheit auszutauschen. Dies kann mit Hilfe vorliegend nicht näher dargestellter Wartungsöffnungen bzw. Aufnahmebereiche der Beduftungsvorrichtung 1 realisiert werden.

Der Pumpzerstäuberkopf 6 weist an seinem in Fig.1 oben liegenden Bereich ein Betätigungsfeld 11 auf. Über das Betätigungsfeld 11 wird eine im Pumpzerstäuberkopf 6 befindliche mechanische Flüssigkeitspumpe (vorliegend nicht dargestellt) betätigt. Dies hat zur Folge, dass ein Duftmittelnebel 13 aus der Austrittsöffnung 12 des Pumpzerstäuberkopfs 6 austritt. Der Duftmittelnebel 13 vermischt sich mit der durch den Luftkanal 7 hindurch strömenden Luft A, sodass sich ein Luft-Duftmittel-Gemisch ergibt, welches an der Ausblasöffnung 10 austritt. Um eine möglichst rasche, vollständige und gleichmäßige Durchmischung von Luft und Duftmittelnebel 13 zu bewirken, ist auf der Oberseite des Duftmittelbehälters 5 eine Prallplatte 14 angeformt. Bei der Prallplatte 14 kann es sich beispielsweise um eine Kunststoffplatte handeln. Es ist im Übrigen möglich, die Prallplatte 14 und den Duftmittelbehälter 5 gleichzeitig durch einen Kunststoffspritzgussvorgang herzustellen. Wie in Fig.1 dargestellt bewirkt die Prallplatte 14 eine Verwirbelung B der Luftströmung, was zu einer verstärkten Durchmischung von Luft und Duftmittelnebel 13 führt.

Es wird darauf hingewiesen, dass ein Teil des Duftmittelnebels 13 auf der Prallplatte 14 auftreffen kann. Jedoch werden die dort landenden Duftmitteltröpfchen zumindest nach einer gewissen Zeit verdunsten, und schlussendlich zu einer Beduftung des Kraftfahrzeuginnenraums führen. Da dies eine gewisse Zeit dauern kann, ist es jedoch möglich, dass es zu einer Verkeimung im Bereich der Prallplatte 14 kommt. Von daher ist es sinnvoll, die Prallplatte 14 integral mit der Duftmittelkartusche 2 auszubilden, sodass bei einem Wechsel der Duftmittelkartusche 2 auch die Prallplatte 14 mit ausgetauscht wird, und auf diese Weise die Verunreinigungen oder die Keimzahl verringert wird. Des Weiteren lässt sich ungewollte Vermischung eines, nach einem Wechsel der Kartusche, neuen Duftstoffes mit dem vorher eingesetzten Duftstoff besser vermeiden, so dass es nicht zu einer Verfälschung der Duftnote kommt.

Bei der in Fig.1 dargestellten Beduftungsvorrichtung 1 erfolgt die Betätigung des Betätigungsfelds 11 über einen Stößel 15. Der Stößel 15 wird von einer Pleuelanordnung 16 in eine zyklische Verschiebebewegung C versetzt. Diese zyklische Verschiebebewegung C führt zu einem entsprechenden Austrag von Duftmittehebel 13. Die Pleuelanordnung 16 wiederum wird von einem Elektromotor 17 angetrieben. Je nachdem, ob der Elektromotor 17 im Dauerbetrieb betrieben, oder nur kurzzeitig betrieben wird, kommt es zu einer dauerhaften, intervallartigen Beduftung der aus der Ausblasöffnung 10 austretenden Luft, oder zu einer nur kurzzeitigen Beduftung der aus der Ausblasöffnung 10 austretenden Luft. Die Ansteuerung des Elektromotors 17 erfolgt durch eine elektronische Steuerung 18. Die elektronische Steuerung 18 steuert darüber hinaus die Funktion des Gebläses 8. Darüber hinaus kann die Steuerung 18 Eingabesignale verarbeiten. Bei den Eingabensignalen kann es sich beispielsweise um Steuerkommandos eines Benutzers händeln. Auch können als Eingabesignal die Ausgabewerte unterschiedlicher Sensoren (beispielsweise Temperatursensoren, Duftseosoren usw.) verwendet werden.

In Fig.1 ist aus zeichnerischen Gründen lediglich eine Leseschleife 19 eingezeichnet. Mit Hilfe der Leseschleife 19 kann auf drahtlosem Weg die in einem RFID-Chip 20 (RFID Radio Frequency Identification) gespeicherte Information ausgelesen werden.

Der RFID-Chip 20 ist in einem Bereich des Duftmittelbehälters 5 der Duftmittelkartusche 2 eingesetzt. In ihm sind beispielsweise Informationen zur Duftart des im Duftmittelbehälter 5 befindlichen Duftmittels, zum Herstellungsdatum, zum Verfallsdatum, Anwendungshinweise (beispielsweise Hinweise zum Mengenverhältnis von Duftmittel zu Luft) gespeichert. Möglich ist es auch, dass der RFID-Chip 20 mit einem Sensor in Verbindung steht, der den aktuellen (Rest-) Vorrat an Duftmittel in Duftmittelbehälter 5 misst.

Die von der Leseschleife 19 aus dem RFID-Chip 20 ausgelesenen informationen werden an die elektronische Steuerung 18 weitergegeben. Diese nutzt die gewonnenen Informationen beispielsweise für die Ansteuerung der Beduftungsvorrichtung 1. Darüber hinaus kann auch der Benutzer über ein Display über den Befüllungsstand des Duftmittelbehälters 5 informiert werden und gegebenenfalls gesondert auf einen in Kürze erforderlich werdenden Tausch der Duftmittelkartusche 2 hingewiesen werden.

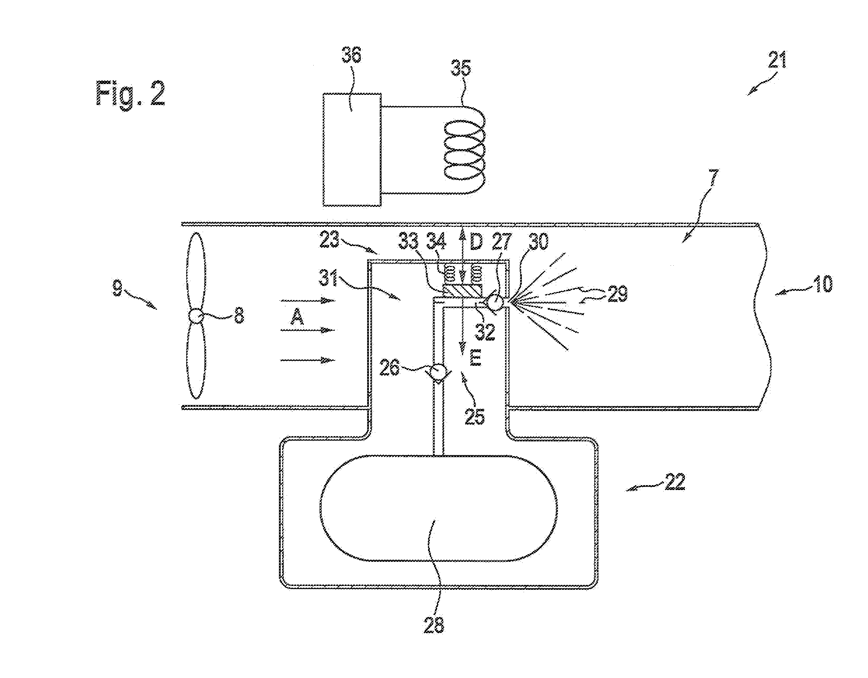

In Fig. 2 ist ein zweites Ausführungsbeispiel für eine Beduftungsvorrichtung 21 mit einer Duftmittelkartusche 22 dargestellt. Fig.2 stellt die Beduftungsvorrichtung 21 in einem schematischen Querschnitt dar.

Analog zur in Fig.1 dargestellten Beduftungsvorrichtung 1 ist auch bei der vorliegend dargestellten Beduftungsvorrichtung 21 ein Luftkanal 7 mit einer Ansaugöffnung 9 und einer Ausblasöffnung 10 vorgesehen. In Luftkanal 7 befindet sich ebenfalls ein Gebläse 8, mit dem Luft A durch den Luftkanal 7 gefördert werden kann.

Der obere Teil 23 der Duftmittelkartusche 22 befindet sich im Luftkanal 7. Im oberen Teil 23 der Duftmittelkartusche 22 ist eine magnetisch angesteuerte Pumpe 25 ausgebildet. Die magnetisch angesteuerte Pumpe 25 fördert Duftmittel, welches sich in einem Vorratsvolumen 28 der Duftmittelkartusche 22 befindet, über eine Ausgabeöffnung 30 als Duftmittelnebel 29 in den LuftStrom A, wo es zu einer Durchmischung von Luft und Duftmittelnebel 29 kommt. Das Vorratsvolumen 28 sowie die Ausgabeöffnung 30 stehen jeweils über ein Rückschlagventil 26, 27 mit dem eigentlichen Pumpenbereich 31 in Verbindung. Der Pumpenbereich 31 weist ein variables Volumen 32 auf. Das variable Volumen 32 kann über eine Magnetplatte 33 varilert werden. Die Magnetplatte 33 kann über ein magnetisches Feld in Pfeilrichtung D bewegt werden, wodurch sich das Volumen 32 des Pumpenbereichs 31 vergrößert. Wird das Magnetfeld abgeschaltet, so wird die Magnetplatte 33 durch Rückstellfedern 34 in Pfeilriclitung E zurückbewegt. Dadurch verkleinert sich das variable Volumen 32 des Pumpenbereichs 31. Die Änderung des variablen Volumens 32 führt in Verbindung mit den Rückschlagventilen 26, 27 zu einer Förderung des Duftmittels. Das Magnetfeld, das die Bewegung der Magnetplatte 33 bewirkt, wird durch eine Magnetspule 35 erzeugt. Im inneren der Magnetspule 35 kann zur Verstärkung des magnetischen Felds ein Kern aus einem weichmagnetischen Material vorgesehen werden. Die Magnetspule 35 wird von einer Stromversorgungsvorrichtung 36 mit elektrischem Strom versorgt.

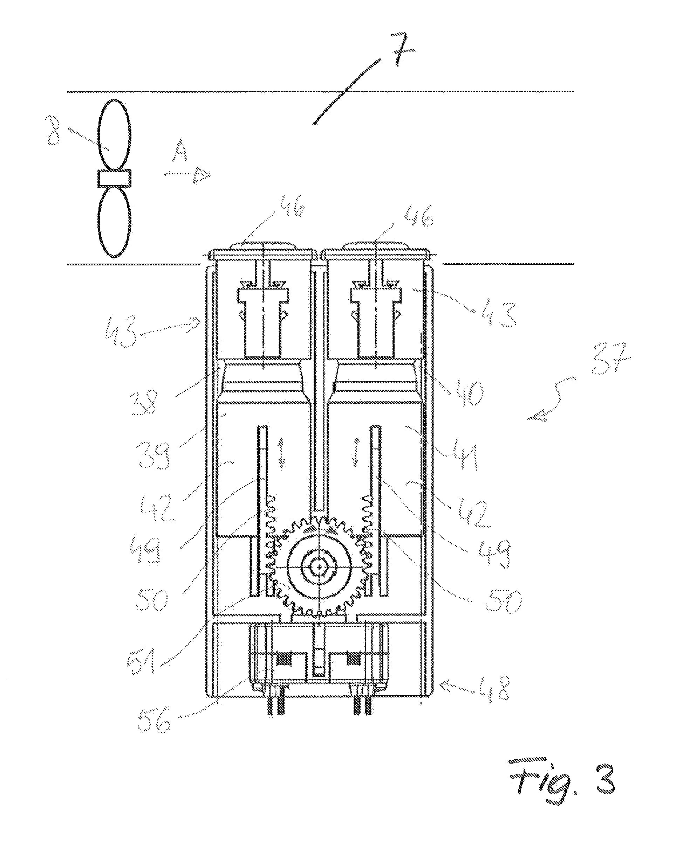

In Fig.3 ist ein drittes Ausführungsbeispiel für eine Beduftungsvorrichtung 37 dargestellt, welche mit Aufnahmeräumen 38, 40 für zwei Duftmittelkartuschen 39, 41 versehen ist. In Fig. 3 ist die Beduftungsvorrichtung 37 in einer schematischen Draufsicht von oben dargestellt.

Die Beduftungsvorrichtung 37 mündet analog zu den in den Fig. 1 und 2 dargestellten Austührungsbeispielen ebenfalls in einen Luftkanal 7, in dem ebenfalls ein Gebläse 8 vorgesehen ist, mit dem Luft A durch den Luftkanal 7 gefördert werden kann.

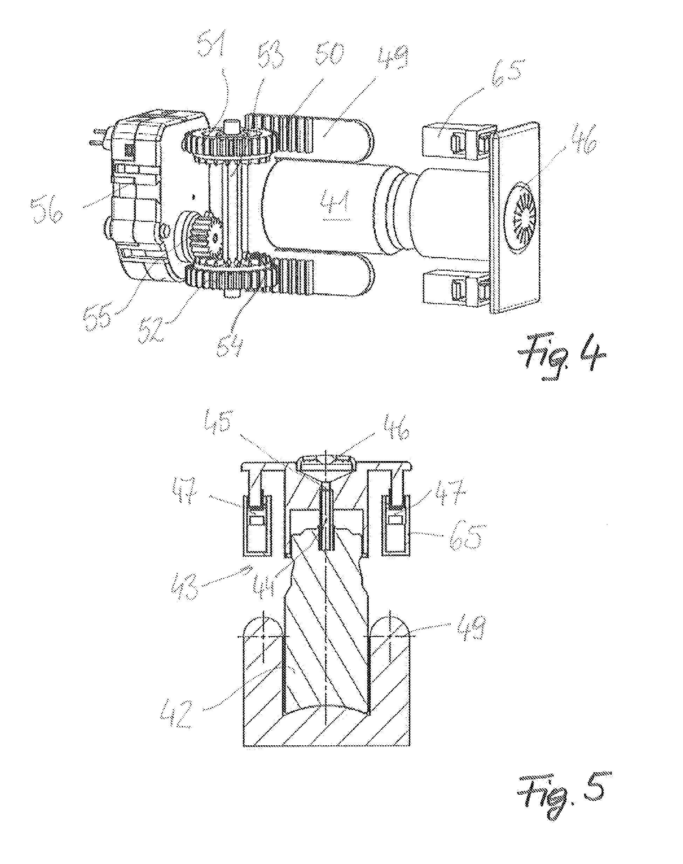

Die Duftmittelkartuschen 39, 41 sind als Druckkartuschen ausgebildet. Ein möglicher Aufbau einer derartigen Duftmittelkartusche 39, 41 ist in Fig. 5 vergrößert dargestellt. Die Duftmittelkartuschen 39, 41 weisen ein Behälterteil 42 auf, in dem das jeweilige Duftmittel unter Druck abgefüllt ist, sowie ein Kopfteil 43 auf. Wird das Kopfteil 43 und das Behälterteil 42 der Duftmittelkartusche 39, 41 aufeinander zu bewegt, so wird ein Ausgabeventil (vorliegend nicht dargestellt) geöffnet, und das im Duftmittelbehälter 42 vorhandene Duftmittel der Duftmittelkartusche 39, 41 strömt über das Ausgaberohr 44 zum Zerstäuber 45. Das im Zerstäuber 45 zerstäubte Duftmittel tritt über die Ausströmkappe 46 der Duftmittelkartusche 39, 41 in den Luftstrom A des Luftkanals 7 aus und vermischt sich dort mit der vom Gebläse 8 durch den Luftkanal 7 geförderten Luft. Wie insbesondere Fig. 5 entnommen werden kann, sind am Kopfteil 43 der Duftmittelkartusche 39 Rasthaken 47 vorgesehen, die in entsprechende Haltebuchsen 65 der Beduftungsvorrichtung 37 eingeführt werden können, und dort clipsartig verrasten. Die Haltebuchsen 65 der Beduftungsvorrichtung 37 und die Haltenasen 47 sind so aufeinander angepasst, dass diese ein sogenanntes Push-Push-Verrastungssystem bilden. Das heißt, dass die Duftmittelkartusche 39 durch Eindrücken des Kopfbereichs 43 in den Aufnahmebereich 38, 40 der Beduftungsvorrichtung 37 arretiert wird. Wird anschließend nochmals ein Druck auf den Kopfbereich 43 der Duftmittelkartusche 39, 41 ausgeübt, so löst sich die Arretierung aus Haltehaken 47 und Haltebuchse 65 der Beduftungsvorrichtung 37 wieder.

Die Betätigung der Duftmittelkartuschen 39, 41 (genauer der Durtmittelbehälter 42 der Duftmittelkartuschen 39, 41 erfolgt durch eine Aktuatorvorrichtung 48, die an der dem Luftführungskanal 7 abgewandten Seite der Beduftungsvorrichtung 37 ausgebildet ist.

Die Aktuatorvorrichtung 48 weist für jede der beiden Duftmittelkartuschen 39, 41 jeweils eine U-förmig ausgebildete Halteklammer 49 auf, die jeweils Teile des Duftmittelbehälters 42 der jeweiligen Duftmittelkartusche 39, 41 umgreifen (vgl. insbesondere Fig. 4). Jeweils an einer Seite sind die Schenkel der Halteklammer 49 mit einer Verzahnung 50 versehen. In die Verzahnung 50 der Halteklammern 49 greift jeweils ein mittig angeordnetes Zahnrad 51, 52 ein. Die beiden Zahnräder (oberes und unteres Zahnrad) 51, 52 sind durch eine Verbindungsachse 53 drehfest miteinander verbunden. Am unteren Zahnrad 52 ist zusätzlich auf einer Stirnseite des Zahnrads 52 eine radial ausgerichtete Verzahnung 54 vorgesehen, in die die Verzahnung eines Antriebszahnrads 55 eines Stellmotors 56 kämmt. Durch eine entsprechende Betätigung des Stellmotors 56 wird somit der Duftbehälterbereich 42 einer der beiden Duftmittelkartuschen 39, 41 in den dazu korrespondierenden Kopfbereich 43 hinein gedrückt, und dadurch die Duftmittelkartusche 39, 41 betätigt. Dementsprechend tritt aus der Ausströmkappe 46 der entsprechenden Duftmittelkartusche 39, 41 eine gewisse Menge an Duftmittel aus.

Durch den Aufbau der Aktuatoreinrichtung 48 ist sichergestellt, dass durch eine Ansteuerung des Stellmotors 56 stets nur ein Duftmittel (falls überhaupt) abgegeben werden kann. Dadurch wird eine Vermischung von zwei unterschiedlichen Düften, die in der Kombination einen befremdlichen Mischgeruch erzeugen könnten, von vornherein vermieden.

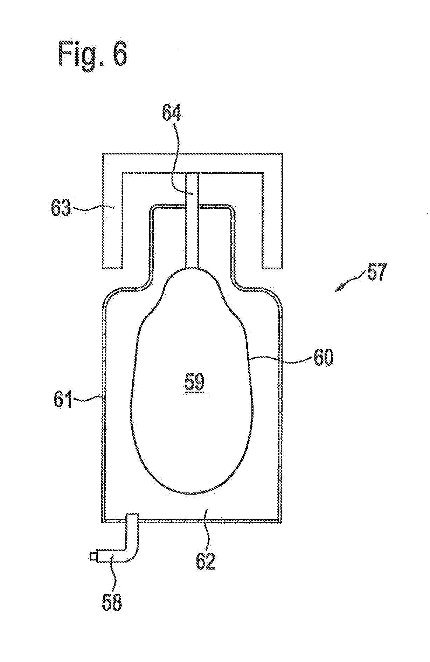

In Fig. 6 ist schließlich noch in einem schematischen Querschnitt skizziert, wie eine Duftmittelkartusche 57 zwar luft- oder keimdicht ausgeführt sein kann, aber dennoch über einen Druckluftversorgungsanschluss 58 mit externer Druckluft beaufschlagt werden kann.

Das Duftmittel 59 befindet sich in einem elastisch verformbaren Duftmittelsack 60. Der Duftmittelsack 60 ist in die Duftmitteldose 61 eingehängt. Der Druckluftanschluss 58 mündet in den Zwischenraum 62, der sich zwischen Duftmittelsack 60 und Duftmittelbehälter 61 befindet. Steht dieser Zwischenraum 62 unter Druck, so steht auch das im Duftmittelsack 60 befindliche Duftmittel 59 unter Druck. Wird nunmehr der Kopfbereich 63 und der Duftmittelbehälter 61 der Duftmittelkartusche 57 aufeinander zu bewegt, so öffnet ein Verschlussventil (vorliegend nicht dargestellt) und das Duftmittel 59 tritt über einen Auslasskanal 64 aus der Duftmittelkartusche 57 aus.

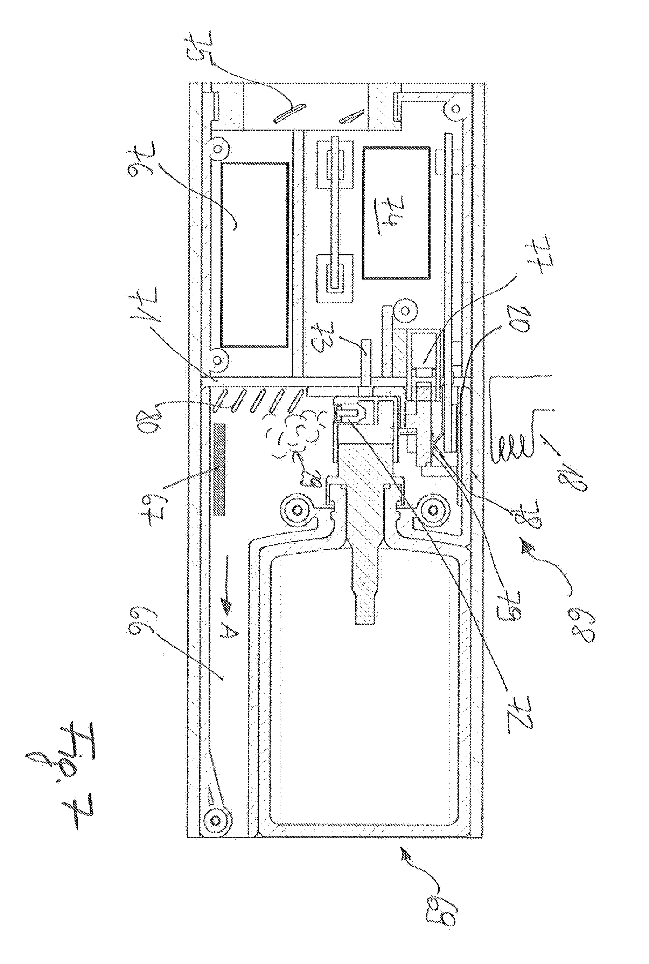

In Fig. 7 ist ein weiteres mögliches Ausführungsbeispiel für eine Beduftungsvorrichtung 68 dargestellt, in der sich eine dazu passend ausgebildete Duftmtttelkartuscheneinheit 69 befindet. Die Beduftungsvorrichtung 68 ist im schematischen Querschnitt dargestellt. Die Beduftungsvorrichtung 68 weist im vorliegend dargestellten Ausführungsbeispiel zwei Aufnahmebereiche 71 für jeweils eine Duftmittelkartuscheneinheit 69 auf. Aufgrund der Querschnittsdarstellung ist in Fig. 7 jedoch nur eine Duftmittelkartuscheneinheit 69 zu erkennen. Die Duftmittelkartuscheneinheit 69 ist mit Hilfe einer Push-Push-Verrasteinheit 77 im dazu korrespondierenden Aufnahmebereich 71 verrastet. Die elektrische Kontaktierung der Duftmittelkartuscheneinheit 69 erfolgt über elektrische Kontaktzungen 78 und dazu korrespondierend ausgebildete elektrische Kontaktflächen 79. Vorzugsweise sind die elektrischen Kontaktflächen 79 in Zusammenhang mit der Duftmittelkartuscheneinheit 69 ausgebildet.

In der Duftmittelkartuscheneinheit 69 ist die eigentliche Duftmittelkartusche 70 angeordnet, die von ihrem grundsätzlichen Aufbau her den in den Fig. 3 bis 6 dargestellten Duftmittelkartuschen 39, 41, 57 ähneln kann. Der Sprühkopf 72 der Duftmittelkartusche 70 ist so ausgebildet, dass der Duftmittelnebei 29 seitlich in den Luftstrom A (Luftdurchströmung in Pfeilrichtung A) eingetragen wird. Der Sprühkopf 72 wird durch einen in der Beduftungsvorrichtung 68 vorgesehenen Betätigungsstößel 73 betätigt. Der Betätigungsstößel 73 wird von einem Elektromotor 74 angetrieben. Der Sprühkopf 72 ist dabei mit einer Verdrehsicherung ausgestattet, damit der Duftmittelnebel 29 nicht in die falsche Richtung freigesetzt wird. Die Verdrehsicherung kann beispielsweise durch einen Vorsprung realisiert werden, der in einer dazu korrespondierenden Führungsnut gleitet. Der Vorsprung kann beispielsweise im Hauptkörper der Duftmittelkartusche vorgesehen werden, während sich die Führungsnut im Sprühkopf 72 befindet.

Der Luftstrom A wird von einem Gebläse 75 erzeugt. Anschließend trifft der Luftstrom A auf eine beweglich angeordnete Luftleitklappe 76. Die Luftleitklappe 76 lenkt den Luftstrom A so ab, dass lediglich einer der Aufnahmebereiche 71 der Beduftungsvorrichtung 68 - und damit lediglich eine Duftmittelkartuscheneinheit 69 - vom Luftstrom A durchströmt wird. Die Stellung der Luftleitklappe 76 kann mechanisch (beispielsweise durch eine mechanische Verbindung mit einem Betätigungselement für den Benutzer) oder elektrisch (beispielsweise durch einen Stellmotor, der von einer elektronischen Steuerung angesteuert wird) verändert werden.

Anschließend tritt der Luftstrom A über ein Schutzgitter 80 in einen Luftkanalbereich 66, der in der Duftmittelkartuscheneinheit 69 vorgesehen ist, ein. Das Schutzgitter 80 ist dabei bevorzugt gemeinsam mit der Duftmittelkartuscheneinheit 69 ausgebildet, kann aber zusätzlich oder alternativ auch gemeinsam mit der Beduftungsvorrichtung 68 ausgebildet werden. Der in der Duftmittelkartuscheneinheit 69 vorgesehene Luftkanalbereich 66 wird bei einem Wechsel der Duftmittelkartuscheneinheit 69 mitgetauscht, so dass Duftmittelreste, die sich gegebenenfalls auf den Wänden des Luftkanalbereichs 66 ablagern können, bei einem Wechsel der Duftmittelkartuscheneinheit 69 zwangsläufig entfernt werden.

Gegenüber dem Sprühkopf 72 ist an der Wand des Luftkanalbereichs 66 der Duftmittelkartuscheneinheit 69 zusätzlich ein elektrisches Heizfeld 67 angeordnet. Das Heizfeld 67 befindet sich dabei in einem Bereich, in dem sich einzelne Tröpfchen des Duftmittelnebel 29 bevorzugt niederschlagen können. Mit Hilfe des Heizfeld 67 werden sich dort gegebenenfalls ablagernde Duftmitteltröpfchen rasch wieder verdampft. Die Temperatur des Heizfelds 67 liegt im Betrieb üblicherweise bei 40° bis 50°C, um einen guten Kompromiss zwischen rascher Verdampfung von sich niederschlagendem Duftmittel und möglichst geringer Duftveränderung durch Erhitzung des Duftmittels zu erzielen.

Ähnlich dem in Fig. 1 dargestellten Ausführungsbeispiel einer Beduftungsvorrichtung 1 weist auch beim vorliegend in Fig. 7 dargestellten Ausführungsbeispiel einer Beduftungsvorrichtung 68 die Duftmittelkartuscheneinheit 69 einen RFID-Chip 20 auf, auf dem diverse Informationen, wie beispielsweise Informationen betreffend die Duftrichtung der Duftmittelkartusche 70, den Füllstand der Duftmittelkartusche 70 oder das Herstellungsdatum der Duftmittelkartusche 70 gespeichert sind. Die auf dem RFID-Chip 20 gespeicherten Informationen können auch hier mittels einer Leseschleife 19 auf drahtlosem Wege ausgelesen werden. Umgekehrt ist es aber auch möglich, dass mittels der Leseschleife 19 Informationen auf den RFID-Chip 20 geschrieben werden können.

## Patentansprüche

1. Duftmittelvorratsvorrichtung (2, 22, 37, 69), aufweisend wenigstens eine Duftmittelbehältereinrichtung (5, 28, 39, 41, 57, 70) und wenigstens eine Duftmittelaustragseinrichtung (6, 25, 46, 64, 72), **gekennzeichnet durch** wenigstens eine Koppelungseinrichtung (15, 49, 73) zur Ankopplung an wenigstens eine Aktuatoreinrichtung (17, 35, 56, 74).

2. Duftmittelvorratsvorrichtung (2, 22, 37, 69), insbesondere Duftmittelvorratsvorrichtung (2, 22, 37, 69) nach dem Oberbegriff von Anspruch 1, bevorzugt Duftmittelvorratsvorrichtung (2, 22, 37, 69) nach Anspruch 1, **gekennzeichnet durch** zumindest ein Informationsspeichermittel (20), welches insbesondere als elektronisches Informationsspeichermittel (20) ausgebildet ist.

3. Duftmittelvorratsvorrichtung (2, 22, 37, 69) nach Anspruch 1 oder 2, **dadurch gekennzeichnet, dass** wenigstens eine Koppelungseinrichtung zumindest teilweise als mechanische Koppelungseinrichtung (15, 49, 73), zumindest teilweise als magnetische Koppelungseinrichtung (35), zumindest teilweise als elektrostatische Koppelungseinrichtung, zumindest teilweise als Fluiddruckeinrichtung und/oder zumindest teilweise als akustische Koppelungseinrichtung ausgeführt ist.

4. Duftmittelvorratsvorrichtung (2, 22, 37, 69) nach einem der vorangehenden Ansprüche, **dadurch gekennzeichnet, dass** die Duftmittelvorratsvorrichtung (2, 22, 37, 69), insbesondere zumindest eine Duftmittelbehältereinrichtung (5, 28, 39, 41, 57, 70), luftdicht ausgeführt ist

5. Duftmittelvorratsvorrichtung (2, 22, 37, 69) nach einem der vorangehenden Ansprüche, **dadurch gekennzeichnet, dass** die Duftmittelvorrats-vorrichtung (2, 22, 37, 69), insbesondere zumindest eine Duftmittelbehältereinrichtung (5, 28, 39, 41, 57, 70) und/oder zumindest eine Duftmittelaustragseinrichtung (6, 25, 46, 64, 72) zumindest teilweise wieder befüllbar und/oder zumindest teilweise rezyklierbar ausgeführt ist.

6. Duftmittelvorratsvorrichtung (2, 22, 37, 69) nach einem der vorangehenden Ansprüche, **dadurch gekennzeichnet, dass** zumindest eine Duftmittelaustragseinrichtung (6, 25, 46, 64, 72) zumindest eine Zerstäubereinrichtung (12, 30, 45, 72) und/oder eine Pumpenzerstäubereinrichtung (6, 72) aufweist.

7. Duftmittelvorratsvorrichtung (2, 22, 37, 69) nach einem der vorangehenden Ansprüche, **gekennzeichnet durch** wenigstens ein Verrastmittel (47, 65, 77) zur Verrastung in einem Aufnahmebereich (38, 40, 71) für die Duftmittelvorratsvorrichtung (2, 22, 37, 69).

8. Duftmittelvorratsvorrichtung (2, 22, 37, 69) nach einem der vorangehenden Ansprüche, **gekennzeichnet durch** wenigstens einen integral ausgebildeten Luftkanalbereich (66).

9. Duftmittelvorratsvorrichtung (2, 22, 37, 69) nach einem der vorangehenden Ansprüche, **gekennzeichnet durch** wenigstens eine Duftmittelbehandlungseinrichtung (11, 67) für ausgetragenes Duftmittel, welche insbesondere als Heizeinrichtung (67) und/oder als Luftverwirbelungseinrichtung (11) ausgebildet ist.

10. Beduftungsvorrichtung (1, 21, 37, 68), insbesondere Beduftungsvorrichtung für ein Fahrzeug, vorzugsweise Beduftungsvorrichtung für ein Kraftfahrzeug, **gekennzeichnet durch** wenigstens eine Duftmittelvorratsvorrichtung (2, 22, 37, 69) nach einem der Ansprüche 1 bis 9, wobei zumindest eine Duftmittelvorratsvorrichtung (2, 22, 37, 69) vorzugsweise austauschbar ausgeführt ist.

11. Beduftungsvorrichtung (1, 21, 37, 68), insbesondere Beduftungsvorrichtung (1, 21, 37, 68) nach dem Oberbegriff von Anspruch 10, bevorzugt Beduftungsvorrichtung (1, 21, 37, 68) nach Anspruch 10, **gekennzeichnet durch** wenigstens ein Informationsübermittlungsmittel (19), welches insbesondere als elektronisches Informationsübermittlungsmittel (19) ausgebildet ist.

12. Beduftungsvorrichtung (1, 21, 37, 68) nach Anspruch 10 oder 11, **gekennzeichnet durch** wenigstens einen Aufnahmeraum (38, 40, 71) und/oder wenigstens ein Verrastmittel (47, 65, 77) zur Aufnahme wenigstens einer Duftmittelvorratsvorrichtung (2, 22, 37, 69).

13. Beduftungsvorrichtung (1, 21, 37, 68) nach einem der Ansprüche 10 bis 12, **gekennzeichnet durch** wenigstens eine Luftbeeinflussungseinrichtung, insbesondere wenigstens eine Luftfördereinrichtung (8), wenigstens eine Lufttemperaturänderungseinrichtung und/oder wenigstens eine Luftverwirbelungseinrichtung (14).

14. Beduftungsvorrichtung (1, 21, 37, 68) nach einem der Ansprüche 10 bis 13, **dadurch gekennzeichnet, dass** die zumindest eine Aktuatorvorrichtung (17, 35, 48) zumindest teilweise als Motoreinrichtung (17, 56, 74), zumindest teilweise als Magneteinrichtung (35), zumindest teilweise als elektrostatische Einrichtung, zumindest teilweise als fluiddruckerhöhende Einrichtung und/oder zumindest teilweise als Bimetalleinrichtung ausgebildet ist.

15. Beduftungsvorrichtung (1, 21, 37, 68) nach einem der Ansprüche 10 bis 14, **gekennzeichnet durch** eine Mehrzahl von Aufnahmeräumen (38, 40, 71) zur Aufnahme von Duftmittelvorratsvorrichtungen (5, 28, 39, 41, 57, 69).
